# EUROPÄISCHE PATENTSCHRIFT

(11) **EP 0 828 747 B1**
(45) Veröffentlichungstag und Bekanntmachung des Hinweises auf die Patenterteilung: **22.05.2002**
(21) Anmeldenummer: 96905784.3
(22) Anmeldetag: 23.02.1996
(51) Int. Cl.: C07H 3/06

(54) **VERFAHREN ZUR HERSTELLUNG VON ALKYLOLIGOGLUCOSIDEN MIT HOHEM OLIGOMERISIERUNGSGRAD**
PROCESS FOR PREPARING ALKYLOLIGOGLUCOSIDES WITH A HIGH DEGREE OF OLIGOMERIZATION
PROCEDE DE PREPARATION D'ALKYLOLIGOGLUCOSIDES A DEGRE ELEVE D'OLIGOMERISATION

(30) Priorität: 26.05.1995 DE 19519384
(43) Veröffentlichungstag der Anmeldung: 18.03.1998
(73) Patentinhaber: Cognis Deutschland GmbH & Co. KG, 40589 Düsseldorf (DE)
(72) Erfinder: WEUTHEN, Manfred, D-42697 Solingen (DE)
(86) Internationale Anmeldenummer: EP9600752
(87) Internationale Veröffentlichungsnummer: WO9637501

(56) Entgegenhaltungen:
- WO-A-93/10133
- WO-A-94/25275
- WO-A-95/01360

## Beschreibung

Die Erfindung betrifft ein Verfahren zur Herstellung von Alkyloligoglucosiden mit hohem DP-Grad (1,6 bis 2,0), bei dem man Alkylpolyglucoside mit niedrigem DP-Grad (< 1,6) einer Nachpolymerisation unterwirft sowie die Verwendung der Verfahrensprodukte als Entschäumer in der maschinellen Flaschenreinigung.

### Stand der Technik

Alkyloligoglykoside, insbesondere Alkyloligoglucoside stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt.1992),** D.Balzer und N.Ripke in **Seifen-Öle-Fette-Wachse 118, 894 (1992)** und B.Brancq in **Seifen-Öle-Fette-Wachse 118, 905 (1992)** genannt werden sollen.

Zur Herstellung von Alkyloligoglucosiden geht man von Fettalkoholen und Glucose oder Glucosesirup aus, die in Gegenwart saurer Katalysatoren acetalisiert werden. Zur Verschiebung des Reaktionsgleichgewichtes auf die Seite der Produkte wird üblicherweise die preisgünstigere Komponente - der Fettalkohol - im Überschuß vorgelegt und das Reaktionswasser kontinuierlich abdestilliert. Im Anschluß wird zur Vermeidung von Folgereaktionen der saure Katalysator neutralisiert und der überschüssige Fettalkohol im Vakuum abdestilliert. Ein solches Verfahrens wird beispielsweise in der **WO 94/025475** beschrieben. Des weiteren sei auf die **WO 93/10133** verwiesen, aus der die Acetalisierung von Glucosesirup mit Fettalkoholen bekannt ist.

Bei der Reaktion treten nun nicht selektiv ein Mol Fettalkohol und ein Mol Glucose zusammen, in Konkurrenz zur Bildung dieser Monoglucoside findet auch eine ebenfalls sauer katalysierte Eigenkondensation der Glucose statt, wobei Polyzucker gebildet werden. Des weiteren reagiert die Glucose mit bereits gebildeten Alkylglucosiden unter Bildung von Oligoglucosiden. In der Folge resultiert ein komplexes Gemisch von Mono-, Di-, Tri-, Tetra- und Oligoglucosiden, das zudem noch beträchtliche Anteile Polyglucose enthält. Neben der Alkylkettenlänge werden die anwendungstechnischen Eigenschaften der Glucoside, wie z.B. Löslichkeit, Kältetrübungspunkt, Schaumstabilität und Verdickbarkeit, auch durch die Homologenverteilung bestimmt, die über eine Verhältniszahl, den sogenannten "degree of polymerization" (DP) beschrieben wird.

Alkyloligoglucoside des Marktes weisen in der Regel einen niedrigen DP im Bereich von 1,2 bis 1,6 auf. Dies hat vor allem technische Gründe, da sich höhere Oligomerisierungsgrade üblicherweise nur dann einstellen lassen, wenn der Fettalkoholüberschuß in der Acetalisierung gering ist; dies ist jedoch wegen der hohen Anfangsviskosität der Mischungen nur eingeschränkt möglich.

Ein weiteres Problem besteht ferner darin, daß ein geringer Fettalkoholüberschuß die Bildung unerwünschter Polyglucose begünstigt und die Farbqualität sowohl der gebleichten als auch der ungebleichten Produkte signifikant abnimmt. Gleichzeitig besteht im Markt jedoch ein Bedürfnis nach Alkyloligoglucosiden mit hohem DP, die den zur Verfügung stehenden niedrigoligomerisierten Handelsprodukten in mancher Hinsicht überlegen sein können.

Die komplexe Aufgabe der Erfindung hat somit darin bestanden, ein Verfahren zur Herstellung von Alkyloligoglucosiden mit hohem Oligomerisierungsgrad zur Verfügung zu stellen, mit dem man gleichzeitig das Problem der Anfangsviskosität der Reaktionsmischungen, des hohen Polyzuckergehaltes und der unzureichenden Farbqualität zu lösen vermag. Im Hinblick auf die teilweise mangelnde Alkalistabilität der Produkte sollten zudem Alkyloligoglucoside zur Verfügung gestellt werden, die in der maschinellen Flaschenreinigung auch unter stark basischen Bedingungen über verbesserte entschäumende Eigenschaften verfügen.

### Beschreibung der Erfindung

Gegenstand der Erfindung ist ein Verfahren zur Herstellung von Alkyloligoglucosiden mit hohem Oligomerisationsgrad, bei dem man
(a) Fettalkohole der Formel **(I)**

   **R**^{**1**}**OH** **(I)**

   in der R¹ für einen Alkylrest mit 6 bis 22 Kohlenstoffatomen steht, und Glucose im molaren Einsatzverhältnis 3 : 1 bis 10 : 1 bei Temperaturen im Bereich von 90 bis 120°C einer sauer katalysierten Acetalisierung unterwirft,
(b) das Reaktionswasser kontinuierlich abdestilliert,
(c) nach Abschluß der Reaktion den sauren Katalysator zu 0 bis 90 Mol-% neutralisiert,
(d) eine solche Menge des nichtumgesetzten Fettalkohols abtrennt, daß das molare Verhältnis des noch verbliebenen Fettalkohols und der Glucose - bezogen auf die Ausgangsmenge - noch 1 : 1 bis 3 : 1 beträgt,
(e) die Reaktionsmischung einer Nachpolymerisation bei Temperaturen im Bereich von 90 bis 120°C unterwirft,
(f) die verbliebene Menge des sauren Katalysator neutralisiert und die verblieben Menge des nichtumgesetzten Fettalkohols abtrennt,

Überraschenderweise wurde gefunden, daß nach Maßgabe des erfindungsgemäßen Verfahrens Alkyloligoglucoside erhalten werden, die einen hohen Oligomerisierungsgrad im Bereich von 1,6 bis 2,0 aufweisen, ohne daß die Produkte durch einen hohen Polyglucosegehalt oder eine unzureichende Farbqualität belastet wären. Zugleich wird durch das vorgeschlagene Verfahren das ansonsten unvermeidbare Problem einer hohen Anfangsviskosität gelöst, so daß hohe Raum-Zeit-Ausbeuten und eine ökonomische Fahrweise möglich werden. Die Erfindung schließt die Erkenntnis ein, daß die nach dem erfindungsgemäßen Verfahren erhältlichen Alkyloligoglucoside gegenüber vergleichbaren Homologen mit niedrigerem DP eine verbesserte Alkalistabilität aufweisen.

### Fettalkohole

Typische Beispiele für Fettalkohole, die im Sinne der Erfindung als Ausgangsstoffe eingesetzt werden können sind Capronalkohol, Caprylalkohol, 2-Ethylhexylalkohol, Caprinalkohol, Laurylalkohol, Isotridecylalkohol, Myristylalkohol, Cetylalkohol, Stearylalkohol, Isostearylalkohol und Behenylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 8 bis 10 bzw. 12 bis 18 Kohlenstoffatomen wie beispielsweise gehärteter Vorlauf-, Kokos-, Palm-, Palmkem- oder Talgfettalkohol.

### Saure Katalysatoren

Als saure Katalysatoren kommen beispielsweise Schwefelsäure, Alkylschwefelsäurehalbester, Alkylbenzolsulfonsäuren wie etwa p-Toluolsulfonsäure oder Dodecylbenzolsulfonsäure und Sulfobernsteinsäure in Frage. Die Einsatzmenge liegt üblicherweise im Bereich von 0,1 bis 5, vorzugsweise 0,5 bis 2 Gew.-% bezogen auf die Einsatzstoffe.

### Acetalisierung

Die Acetalisierung kann in an sich bekannter Weise erfolgen. Üblicherweise legt man den Fettalkohol zusammen mit dem sauren Katalysator vor, dosiert eine Suspension von Glucose in Fettalkohol zu und erhitzt den Ansatz auf die Reaktionstemperatur. Es ist jedoch ebenfalls möglich, zunächst eine homogene Suspension aus Fettalkohol und Glucose herzustellen, die Reaktionsmischung zu erhitzen und dabei eine Lösung des sauren Katalysators in Fettalkohol zuzudosieren. Diese Phase ist unkritisch, so daß auch andere Verfahrensweise in Betracht kommen. Hier seien insbesondere der Einsatz von wäßrigem Glucosesirup, der vor der Acetalisierung in Gegenwart oder Abwesenheit des Fettalkohols entwässert wird oder die Verwendung von sprüh- bzw. heißdampfgetrocknetem Dextrosesirup genannt. Ebenfalls ist es möglich, anstelle einer direkten Umsetzung von Glucose und Fettalkohol den als "Umacetalisierung" bezeichneten Weg zu wählen, der über die Bildung von Butylglucosiden als Zwischenprodukten führt. Die Reaktion wird unterhalb der Karamelisierungstemperatur des Zuckers bei etwa 100 bis 120°C und vorzugsweise unter vermindertem Druck von 0,1 bis 100 mbar durchgeführt. Aus kinetischen Gründen ist die kontinuierliche Entfernung des bei der Kondensation gebildeten Wassers aus dem Ansatz sehr empfehlenswert.

Im Anschluß an die Acetalisierung, vorzugsweise dann, wenn der Restglucosegehalt unter einen Wert von 3 Gew.-% und insbesondere 1 Gew.-% abgesunken ist, findet nach den Verfahren des Stands der Technik die Neutralisation des sauren Katalysators statt. Im Sinne der Erfindung verzichtet man entweder auf diesen Schritt oder führt die Neutralisation nur anteilig, also beispielsweise zu etwa 10 bis 90 Mol-% durch. Als Neutralisationsbasen kommen in erster Linie Alkalihydroxide und/oder Erdalkalioxide, vorzugsweise wäßrige Natriumhydroxidlösung und Magnesiumoxid in Frage.

### Nachpolymerisation

Bis zu diesem Punkt des Verfahrens hat man demnach die Vorteile einer Arbeitsweise mit hohem Fettalkoholüberschuß genutzt. Somit konnte das Problem der hohen Pastenviskosität, der Bildung unerwünscht großer Anteile an Polyglucose sowie der mangelnden Bleichbarkeit umgangen werden; die nun als Zwischenprodukte vorliegenden rohen Alkyloligoglucoside weisen jedoch noch nicht den gewünscht hohen DP auf.

Durch die partielle oder nicht erfolgte Neutralisation des Katalysators hat man jedoch sichergestellt, daß nach Abtrennung eines Teils des Fettalkohols ausreichend H⁺-ionen zur Verfügung stehen, um die Nachpolymerisation zu unterstützen. Hat das Gewichtsverhältnis zwischen Fettalkohol und Glucose zu Beginn der Reaktion noch 3 : 1 bis 10 : 1, vorzugsweise 6 : 1 bis 8 : 1 betragen, wird nun eine solche Menge Fettalkohol im Vakuum abdestilliert, daß - bezogen auf die Ausgangsmenge - ein Verhältnis von 1 : 1 bis 3 : 1, vorzugsweise 1,5 : 1 bis 2,5 : 1 resultiert. Die Nachpolymerisation der Reaktionsmischung unter Bildung von Alkyloligoglucosiden mit einem vergleichsweise höheren Oligomerisierungsgrad kann in einem separaten Schritt unter den vorgenannten Reaktionsbedingungen erfolgen. In der Praxis ist dies jedoch in der Regel nicht nötig, da die Nachpolymerisation schon unter den Bedingungen der partiellen Abtrennung des Fettalkohols stattfindet und die gewünschten Ergebnisse liefert. In Summe wird die Nachpolymerisation solange durchgeführt, bis sich ein DP im Bereich von 1,6 bis 2,0, vorzugsweise 1,7 bis 1,9 eingestellt hat. Die vollständige Neutralisation des Katalysators, die Abtrennung des verbliebenen überschüssigen Alkohols sowie die alkalische Bleiche kann dann in an sich bekannter Weise erfolgen.

### Gewerbliche Anwendbarkeit

Die nach dem erfindungsgemäßen Verfahren erhältlichen Alkyloligoglucoside sind hellfarbig und weisen ein Oligomerisierungsgrad im Bereich von 1,6 bis 2,0 auf. Sie eignen sich zur Herstellung von Wasch-, Spül- und Reinigungsmitteln sowie von Mitteln zur Haar- und Körperpflege, in denen sie in Mengen von 1 bis 50, vorzugsweise 5 bis 35 Gew.-% - bezogen auf die Mittel - enthalten sein können. Wegen ihrer besonderen Alkalistabilität eignen sie sich insbesondere als Entschäumer für die maschinelle Reinigung von Milch- und Bierflaschen.

### Beispiele

**Vergleichsbeispiel V1.** In einer 3-I-Rührapparatur wurde ein Gemisch aus 1964 g (10,5 mol) Dodecanol und 630 g (3,5 mol) Glucose vorgelegt und bei einem verminderten Druck von 20 mbar auf eine Temperatur von 110°C erhitzt. Anschließend wurden innerhalb von 10 min 6,6 g (20 mmol) Dodecylbenzolsulfonsäure zugetropft. Nach einer Reaktionszeit von 9 h wurde die Reaktion durch Zugabe von 1 ml (19 mmol) 50 Gew.-%iger wäßriger Natriumhydroxidlösung und 3,2 g (80 mmol) Magnesiumoxid abgebrochen. Die Mischung wurde in einen Dünnschichtverdampfer überführt und dort bei einem verminderten Druck von 0,1 mbar und einer Sumpftemperatur von 165°C vom überschüssigen Fettalkohol befreit. Es wurden 915 g Produkt erhalten, die bei 90°C in 900 g Wasser gelöst, mit 27 ml 50 Gew.-%iger wäßriger Natriumhydroxidlösung versetzt und nach Zugabe von 38 ml 35 Gew.-%igen Wasserstoffperoxids über einen Zeitraum von 24 h gebleicht wurden. Die Kenndaten des Produktes sind in Tabelle 1 zusammengefaßt (Prozentangaben als Gew.-%).

**Beispiel 1.** Analog Vergleichsbeispiel V1 wurden 2235 g (12 mol) Dodecanol und 360 g (2,0 mol) Glucose bei einem verminderten Druck von 20 mbar auf eine Temperatur von 110°C erhitzt. Anschließend wurden innerhalb von 10 min 4 g (12 mmol) Dodecylbenzolsulfonsäure zugetropft. Nach einer Reaktionszeit von 6 h wurden der Mischung 0,6 mol (7,2 mmol) 50 Gew.-%ige wäßrige Natriumhydroxidlösung zugesetzt und der Druck auf 0,05 mbar abgesenkt. Danach wurden zunächst bei einer Sumpftemperatur von 95°C 1560 g (8,4 mol) Dodecanol abdestilliert. Nach dem Ende der Destillation wurde der Druck auf 2 mbar angehoben und das Reaktionsgemisch eine weitere h bei 105°C gerührt. Der Reaktionsabbruch erfolgte durch Zugabe von 0,34 g (4,1 mmol) 50 Gew.-%iger Natriumhydroxidlösung und 1,9 g (48 mmol) Magnesiumoxid. Die Abtrennung des restlichen Fettalkohols erfolgte bei einem Vakuum von 0,1 mbar und einer Sumpftemperatur von 165°C. Es wurden 608 g Produkt erhalten, das analog V1 gebleicht wurde. Die Kenndaten sind in Tabelle 1 zusammengefaßt.

**Beispiel 2.** Analog Vergleichsbeispiel V1 wurden 2235 g (12 mol) Dodecanol und 360 g (2,0 mol) Glucose bei einem verminderten Druck von 20 mbar auf eine Temperatur von 110°C erhitzt. Anschließend wurden innerhalb von 10 min 4 g (12 mmol) Dodecylbenzolsulfonsäure zugetropft. Nach einer Reaktionszeit von 6 h wurden der Mischung 0,6 mol (7,2 mmol) 50 Gew.-%ige wäßrige Natriumhydroxidlösung zugesetzt und der Druck auf 0,05 mbar abgesenkt. Danach wurden zunächst bei einer Sumpftemperatur von 95°C 1730 g (9,3 mol) Dodecanol abdestilliert. Nach dem Ende der Destillation wurde der Druck auf 2 mbar angehoben und das Reaktionsgemisch eine weitere h bei 105°C gerührt. Der Reaktionsabbruch erfolgte durch Zugabe von 0,34 g (4,1 mmol) 50 Gew.-%iger Natriumhydroxidlösung und 1,9 g (48 mmol) Magnesiumoxid. Die Abtrennung des restlichen Fettalkohols erfolgte bei einem Vakuum von 0,1 mbar und einer Sumpftemperatur von 165°C. Es wurden 596 g Produkt erhalten, das analog V1 gebleicht wurde. Die Kenndaten sind in Tabelle 1 zusammengefaßt.

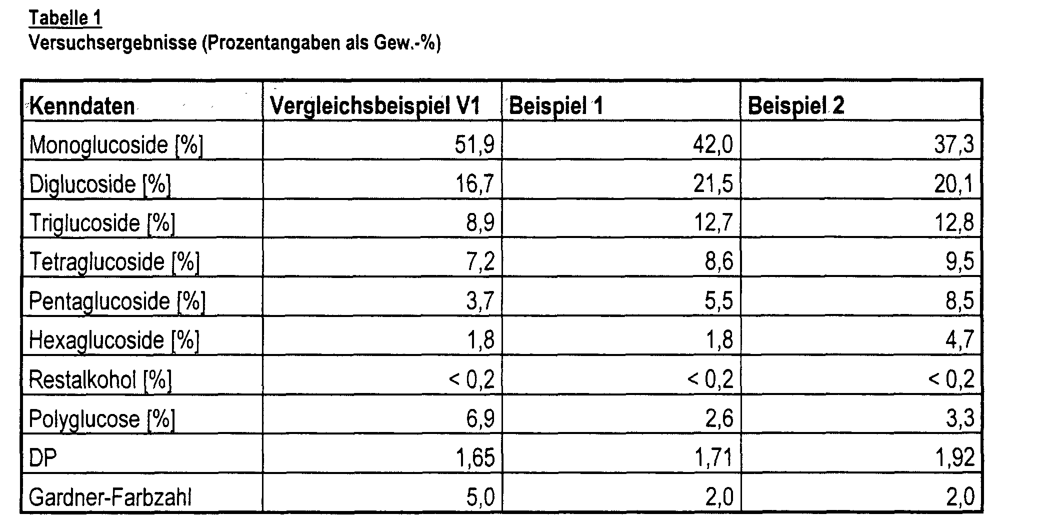

**Anwendungstechnische Beispiele.** In einem doppelwandigen 2-I-Meßzylinder wurden 300 ml einer 1 Gew.-%igen Natriumhydroxidlösung auf 65°C temperiert. Anschließend wurde der jeweils ausgewählte schaumdrückende Zusatzstoff A = Kokosalkyloligoglucosid DP = 1,92 gemäß Beispiel 2 (erfindungsgemäß) bzw. B = Kokosalkyloligoglucosid DP = 1,3 (zum Vergleich) in den nachfolgend angegebenen Mengen zugesetzt. Mit Hilfe einer Laborschlauchpumpe wurde die Flüssigkeit mit einer Umwälzgeschwindigkeit von 4 l/min umgepumpt. Dabei wurde die Prüflösung ca. 5 mm über dem Boden des Meßzylinders mit Hilfe eines Glasrohres, das mit einer Pumpe verbunden war, angesaugt und über ein zweites Glasrohr, das an der 2000-ml-Marke angebracht war, in freiem Fall zurückgeführt. Nach 30 s dosierte man zunächst 1 ml einer 1 Gew.-%igen wäßrigen Lösung von Tetrapropylenbenzolsulfonat-Triethanolammoniumsalz (nachfolgend als "Testschäumer" bezeichnet) in die Flotte und bestimmte nach weiteren 30 s das Volumen, das durch Flüssigkeit und Schaum gebildet wurde. In Abständen von jeweils 1 min wurde weiterer Testschäumer zudosiert und das nach 30 s entstandene Schaum/Flüssigkeits-Volumen bestimmt. Diesen stufenförmigen Zyklus von Zudosierung des Testschäumers und Schaummessung setzte man solange fort, bis die Tensidlösung im Meßzy-linder auf 2000 ml aufgeschäumt war. Die Ergebnisse sind in Tabelle 2 zusammengefaßt.

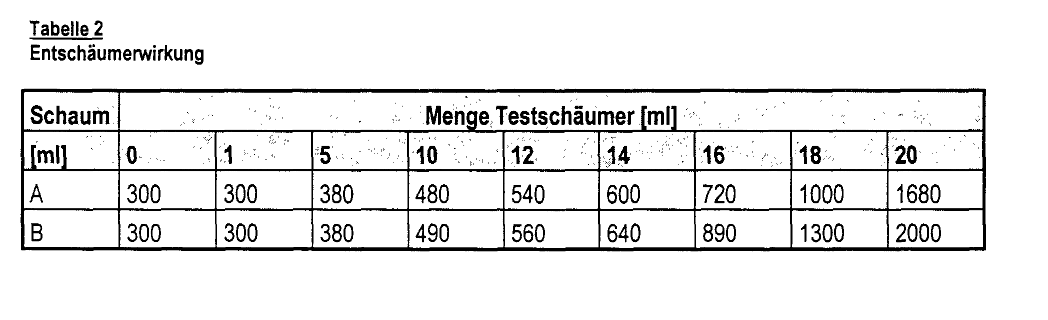

## Patentansprüche

1. Verfahren zur Herstellung von Alkyloligoglucosiden mit hohem Oligomerisationsgrad, bei dem man
(a) Fettalkohole der Formel **(I)**
**R**^{**1**}**OH (I)**
in der R¹ für einen Alkylrest mit 6 bis 22 Kohlenstoffatomen steht, und Glucose im molaren Einsatzverhältnis 3 : 1 bis 10 : 1 bei Temperaturen im Bereich von 90 bis 120°C einer sauer katalysierten Acetalisierung unterwirft,
(b) das Reaktionswasser kontinuierlich abdestilliert,
(c) nach Abschluß der Reaktion den sauren Katalysator zu 0 bis 90 Mol-% neutralisiert,
(d) eine solche Menge des nichtumgesetzten Fettalkohols abtrennt, daß das molare Verhältnis des noch verbliebenen Fettalkohols und der Glucose - bezogen auf die Ausgangsmenge - noch 1:1 bis 3:1 beträgt,
(e) die Reaktionsmischung einer Nachpolymerisation bei Temperaturen im Bereich von 90 bis 120°C unterwirft,
(f) die verbliebene Menge des sauren Katalysator neutralisiert und die verblieben Menge des nichtumgesetzten Fettalkohols abtrennt.

2. Verfahren nach Anspruch 1, **dadurch gekennzeichnet, daß** man saure Katalysatoren ausgewählt aus der Gruppe einsetzt, die gebildet wird von Schwefelsäure, Alkylschwefelsäurehalbestern, Alkylbenzolsulfonsäuren und Sulfobernsteinsäure.

3. Verfahren nach den Ansprüchen 1 und 2, **dadurch gekennzeichnet, daß** man den sauren Katalysator zu 10 bis 90 Mol-% neutralisiert.

4. Verfahren nach den Ansprüchen 1 bis 3, **dadurch gekennzeichnet, daß** man den sauren Katalysator durch Zusatz von Alkalihydroxiden und/oder Erdalkalioxiden neutralisiert.

5. Verfahren nach den Ansprüchen 1 bis 4, **dadurch gekennzeichnet, daß** man die Nachpolymerisation während bzw. nach der partiellen Abtrennung des nichtumgesetzten Fettalkohols durchführt.

6. Verfahren nach den Ansprüchen 1 bis 5, **dadurch gekennzeichnet, daß** man die Nachpolymerisation solange durchführt, bis sich ein DP im Bereich von 1,6 bis 2,0 eingestellt hat.

7. Verwendung der Alkyloligoglucoside erhältlich durch das der Ansprüchen 1 bis 6 als Entschäumer in der maschinellen Flaschenreinigung.

## Claims

1. A process for the production of alkyl oligoglucosides with a high degree of oligomerization, in which
(a) fatty alcohols corresponding to formula (I):
R¹OH (I)
in which R¹ is an alkyl radical containing 6 to 22 carbon atoms, and glucose in a molar ratio of 3:1 to 10:1 are subjected to acid-catalyzed acetalization at temperatures of 90 to 120°C,
(b) the water of reaction is continuously distilled off,
(c) on completion of the reaction, 0 to 90 mole-% of the acidic catalyst is neutralized,
(d) the unreacted fatty alcohol is removed in such a quantity that the molar ratio of the fatty alcohol still remaining to the glucose, based on the starting quantity, is still 1:1 to 3:1,
(e) the reaction mixture is subjected to post-polymerization at temperatures of 90 to 120°C,
(f) the remaining quantity of acidic catalyst is neutralized and the remaining quantity of unreacted fatty alcohol is removed.

2. A process as claimed in claim 1, **characterized in that** acidic catalysts selected from the group consisting of sulfuric acid, alkyl sulfuric acid semiesters, alkyl benzene sulfonic acids and sulfosuccinic acid are used.

3. A process as claimed in claims 1 and 2, **characterized in that** 10 to 90 mole-% of the acidic catalyst is neutralized.

4. A process as claimed in claims 1 to 3, **characterized in that** the acidic catalyst is neutralized by addition of alkali metal hydroxides and/or alkaline earth metal oxides.

5. A process as claimed in claims 1 to 4, **characterized in that** the post-polymerization is carried out during or after partial removal of the unreacted fatty alcohol.

6. A process as claimed in claims 1 to 5, **characterized in that** the post-polymerization is carried out until a DP of 1.6 to 2.0 is established.

7. The use of the alkyl oligoglucosides obtainable by the process claimed in claims 1 to 6 as defoamers in machine bottle washing.

## Revendications

1. Procédé pour la préparation d'alkyloligoglucosides à haut degré d'oligomérisation, dans lequel
(a) on soumet à une acétalisation, catalysée par un acide, des alcools gras de formule (I)
R¹OH (I)
dans laquelle R¹ représente un radical alkyle ayant de 6 à 22 atomes de carbone, et du glucose en un rapport molaire d'utilisation allant de 3:1 à 10:1, à des températures dans la plage de 90 à 120°C,
(b) on élimine en continu l'eau de réaction par distillation,
(c) une fois la réaction terminée, on neutralise à raison de 0 à 90 % en moles le catalyseur acide,
(d) on sépare l'alcool gras n'ayant pas réagi, en une quantité telle que le rapport molaire de l'alcool gras encore restant et du glucose - par rapport à la quantité initiale - va encore de 1:1 à 3:1,
(e) on soumet le mélange réactionnel à une post-polymérisation à des températures dans la plage de 90 à 120°C,
(f) on neutralise la quantité restante du catalyseur acide et on sépare la quantité restante de l'alcool gras n'ayant pas réagi.

2. Procédé selon la revendication 1,
**caractérisé en ce qu'**
on utilise des catalyseurs acides choisis dans le groupe qui est constitué par l'acide sulfurique, les hémiesters d'acides alkylsulfuriques, des acides alkylbenzènesulfoniques et l'acide sulfosuccinique.

3. Procédé selon les revendications 1 et 2,
**caractérisé en ce qu'**
on neutralise le catalyseur acide à raison de 10 à 90 % en moles.

4. Procédé selon les revendications 1 à 3,
**caractérisé en ce qu'**
on neutralise le catalyseur par addition d'hydroxydes de métaux alcalins et/ou d'oxydes de métaux alcalino-terreux.

5. Procédé selon les revendications 1 à 4,
**caractérisé en ce qu'**
on effectue la post-polymérisation pendant ou après la séparation partielle de l'alcool gras n'ayant pas réagi.

6. Procédé selon les revendications 1 à 5,
**caractérisé en ce qu'**
on effectue la post-polymérisation jusqu'à l'établissement d'un DP dans la plage de 1,6 à 2,0.

7. Utilisation des alkyloligoglucosides pouvant être obtenus par le procédé des revendications 1 à 6, en tant qu'antimousse dans le nettoyage de bouteilles à la machine.
